(19) Europäisches Patentamt
European Patent Office
Office européen des brevets

(11) **EP 4 421 752 A1**

(12) **EUROPEAN PATENT APPLICATION**

(43) Date of publication:
**28.08.2024   Bulletin 2024/35**

(21) Application number: **24158894.6**

(22) Date of filing: **21.02.2024**

(51) International Patent Classification (IPC):
**G06V 10/26** $^{(2022.01)}$       **G06V 10/82** $^{(2022.01)}$

(52) Cooperative Patent Classification (CPC):
**G06V 10/82; G06T 7/10; G06V 10/26;**
G06V 2201/03

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.02.2023   US 202363486404 P**

(71) Applicant: **Canon Medical Systems Corporation
Otawara-shi, Tochigi 324-8550 (JP)**

(72) Inventors:
• **Schrempf, Patrick
  Edinburgh, EH6 5NP (GB)**
• **Mackinnon, Hamish
  Edinburgh, EH6 5NP (GB)**
• **O'Neil, Alison
  Edinburgh, EH6 5NP (GB)**

(74) Representative: **Marks & Clerk LLP
15 Fetter Lane
London EC4A 1BW (GB)**

(54) **MEDICAL IMAGE PROCESSING APPARATUS AND METHOD**

(57)     A medical image processing apparatus (2) comprises processing circuitry (12) configured to:
receive medical image data for processing;
receive text data including clinical information relating to the medical image data;
specify based on the text data a target processing region of the medical image data or of a space derived from the medical image data; and
process at least the specified target processing region of the medical image data or of the space derived from the medical image data.

EP 4 421 752 A1

Fig. 1

**Description**

**Field**

Embodiments described herein relate generally to a method and apparatus for processing data, for example for training and using a model to provide image segmentation.

**Background**

Scan requests for medical imaging may contain clinical information about the patient, including the patient's history of symptoms. When viewing radiology scans in a clinical setting, radiologists use the information to determine possible diagnoses. In particular, this information can be important when there are multiple differential diagnoses that cannot be differentiated by the image alone.

It is known to train convolutional neural networks (CNNs) and other machine learning models to process data, for example medical data.

Training of machine learning models can be performed using either supervised or unsupervised techniques, or a mixture of supervised and unsupervised techniques.

It is known to spatially condition the predictions of a convolutional neural network (CNN) on clinical non-image inputs.

In United States Patent Application No. 16/992,466, the contents of which are hereby incorporated by reference in their entirety, a medical image data processing apparatus is provided and includes processing circuitry to receive medical image data in respect of at least one subject; receive non-image data; generate a filter based on the non-image data; and apply the filter to the medical image data, wherein the filter limits a region of the medical image data. Image segmentation, as applied in this example, is the task of pixelwise labelling of different objects within an image. This can be done in two or three dimensions, and serves to spatially identify items of interest, such as pathologies in medical images. CNNs based methods are usually used for image segmentation.

'Instance modulation with spatial dependency' (INSIDE) is a mechanism for spatially conditioning the preconditions of a convolutional neural network on clinical information inputs in order to focus attention of the network on a specific part of the image obtained from the clinical information inputs. INSIDE layers are sandwiched between convolutional layers of the neural network, and create Gaussian attention spatial filters which are applied to the intermediate feature maps

**Summary**

In a first aspect, there is provided a medical image processing apparatus comprising processing circuitry configured to:

  receive medical image data for processing,
  receive text data including clinical information relating to the medical image data,
  specify based on the text data a target processing region of the medical image data or of a space derived from the medical image data,
  process the specified target processing region of the medical image data or of the space derived from the medical image data.

The processing may comprise processing the medical image data with increased attention provided to the specified target processing region.

The space derived from the medical image data may comprise latent space, for example a latent space or an image segmentation model. The target processing region may be not directly corresponding to a region of the image but instead to characteristics of features of the image.

The processing may comprise performing a segmentation process. The segmentation process may comprise segmenting a specified anatomical feature and/or pathology.

The process may comprise applying a main trained machine learning model, for example a main trained neural network, to the medical image data. The main trained machine learning model may be configured to include or receive conditioning information to condition outputs of the main machine learning model such that the outputs depend on both the medical image data and the conditioning information. The conditioning information may comprise non-image data.

The processing circuitry may be configured to apply an auxiliary trained machine learning model, for example an auxiliary trained neural network, to the text data. The specifying of the target processing region may comprise applying the auxiliary trained machine learning model to the text data thereby to specify the target processing region.

The auxiliary trained machine learning model may comprise a text encoder network.

The auxiliary trained machine learning model, for example the text encoder network, may be configured to combine individual token representations of the text data into a latent representation of the text data (e.g. using concatenation or via a neural network).

The text encoder network may be configured to generate embeddings for tokens that represent text of the text data, and to combine (for example concatenate) the embeddings into a vector that is or forms part of the conditioning information input to the main machine learning model.

The auxiliary trained machine learning model may be configured to learn parameters for use as conditioning information to be input to the main machine learning model, for example such that the combination of the text encoding of the conditioning information and the main inputs to the neural network influences the outputs of the main machine learning model.

The processing circuitry may be configured to apply an additional conditioning process, for example to the output of the text encoder or other auxiliary machine learning model, either during training or in use. The additional conditioning process may be such as to ensure or encourage that spatial information in the text data, for example determined according to a ground truth, is represented in, or taken into account by, inputs to and/or outputs from the main machine learning model.

The additional conditioning process may be such as to alter an attention vector, for example representing spatial attention, that is input to or included in the main machine learning model.

The additional conditioning process may represent a shape or other property of a pathology or other feature of interest. The additional conditioning process may comprise or represent a signed distance field or other loss function. The additional conditioning process may provide attention, for example attention vector(s), that is represented by or generated using a signed distance field or other loss function. A function that is used to represent or generate the attention in the conditioning process may be selected based on output from the text encoder.

A plurality of attention shapes may be learned using signed distance fields or other loss functions. A correct attention shape may then be selected (e.g. using a switch function or any other suitable mechanism or process) for example according to the input conditioning.

The training of the text encoder network, or other auxiliary trained machine learning model, may comprise applying a penalty to a loss function in order to train against structured data.

The training of the main machine learning model may comprise applying a penalty to a loss function in order to train against structured data.

The training of the main machine learning model may comprise using at least some counterfactual training data and/or using a counterfactual training technique. The training data may comprise at least some training data that represent counterfactual examples or other property that encourage the neural network or other trained model to use or be more influenced by the conditioning information and/or the output of the auxiliary trained machine learning model.

The text data may comprise at least one radiology report and/or clinician notes or other user notes. The output of the main trained machine learning model may comprise pathology segmentation, for example for use in monitoring change of a pathology over time.

The processing circuitry may be further configured to receive user input, and to alter at least one of spatial attention mechanisms of the main machine learning model in dependence on the user input and/or augment or otherwise modify the text input based on the user input. The processing circuitry may be configured to use the user input in a feedback loop that comprises modifying at least one of input to the auxiliary model, output of the auxiliary model, and/or input to the main model based on the user input and recalculating the output of the main model.

The auxiliary model may be trained or pre-trained on conversational data, e,g, trained to respond to instructions.

The processing circuitry may be configured to input the user input to a further trained machine learning model, which is trained to generate outputs that may be input to the main machine learning model and/or auxiliary machine learning model and/or that may be used to alter the conditioning information. The user input may comprise or represent prompts to provide improved or alternative segmentations or other outputs.

The text encoder or other auxiliary model may provide an attention mechanism and to derive attention parameter(s) to be input to the main model.

The processing circuitry may be configured to apply a further trained machine learning model (for example, at least one attention layer) to the output of the text encoder to extract entities or other features from the text data, to extract encodings from the text encoder output that correspond to the entities or other features, and to use the extracted encodings to condition a further attention process.

In a further aspect, which may be provided independently, there is provided a method of image processing apparatus comprising:

receiving medical image data; receiving text data including clinical information relating to the medical image data; specifying a target processing region of the medical image data based on the text data; and processing at least the specified target processing region of the medical image data.

In a further aspect, which may be provided independently, there is provided a processing apparatus configured to train a main machine learning model and/or an auxiliary machine learning model based on sets of training image data and associated text data,

wherein the training of the main machine learning model comprises training the main machine learning model to perform a segmentation based on medical image data and conditioning information received from the auxiliary machine learning model; and
the training of the auxiliary machine learning model comprises training the auxiliary machine learning model on at least text data to provide conditioning information to the main machine learning model to specify a target processing region.

In a further aspect, which may be provided independently, there is provided a method of training a main machine learning model and/or an auxiliary machine learning model based on sets of training image data and associated text data, the method comprising:

training the main machine learning model to perform a segmentation based on medical image data and conditioning information received from the auxiliary machine learning model; and/or
training the auxiliary machine learning model on at least text data to provide conditioning information to the main machine learning model to specify a target processing region.

In a further aspect, which may be provided independently, there is provided a system for integrating semantic information from text comprising:

a neural network,
a mechanism for integrating conditioning information (for example, FiLM, INSIDE),
a set of inputs,
conditioning data in the form of text,
an auxiliary text encoder network which learns parameters for the conditioning mechanism, for example by combining individual token representations into a latent representation of the text (for example, concatenation or via a neural network) such that the combination of the text encoding of the conditioning data and the main inputs to the neural network influences outputs.

A penalty in a loss function may be applied to the text encoder to train against structured data (e.g. with the use of synthetic data).

A penalty in a loss function may be applied to the conditioning mechanism to train against structured data (e.g. with the use of synthetic data).

The network may be trained with a counterfactual training technique. The data may be augmented via techniques (e.g. "copy, mirror, paste") to create counterfactual examples that encourage the neural network to use the conditioning mechanism.

The additional text input may comprise a prior radiology report and/or an output of the network may comprise a pathology segmentation e.g. for change monitoring.

The system may comprise, or be used in, a feedback loop with a clinician, who can influence the network's spatial attention.

Attention parameters may be derived from and/or coupled with an attention mechanism of the text encoder network.

The text data may comprise or represent spatial information, for example information relevant to, determinative of, or dependent on, location of a pathology or other feature of interest. The text data may comprise or represent a type of pathology, a type of symptom, a shape and/or location and/or type of previous pathologies, and/or a location of a symptom.

A medical imaging procedure may start with a scan request written by a clinician, which contains information about the patient's history such as the patient's symptoms. This scan request may be used by radiologists to determine the possible diagnoses when viewing radiology scans in clinical practice. In particular, this information can be important when there are multiple differential diagnoses that cannot be differentiated by the image alone.

In a further aspect, which may be provided independently, to improve image segmentation scan request text is input into an image segmentation algorithm by extending an existing spatial dependency module (e.g. INSIDE). Free text input may be processed by a natural language processing (NLP) model and may guide the image segmentation model to a certain location or shape in the input data via a learnt attention. The text encoder may be used to extract semantic information that is relevant to different types of symptoms or pathologies. A structured data loss may be used to improve the training of a conditional layer via supervision of the latent representation. Furthermore, for scenarios with limited amounts of available training data, counterfactual data augmentation may be used to improve the model's extraction of salient information from text conditioning input.

Features in one aspect or embodiment may be combined with features in any other aspect or embodiment in any appropriate combination. For example, apparatus features may be provided as method features and vice versa.

**Brief description of the drawings**

Embodiments are now described, by way of non-limiting example, and are illustrated in the following figures, in which:

Figure 1 is a schematic illustration of an apparatus for medical image processing in accordance with an embodiment;
Figure 2 is a schematic representation of a method of medical image processing according to an embodiment;
Figure 3 is a schematic illustrating a method of encoding text in accordance with an embodiment;
Figure 4 is a schematic illustrating a method of medical image processing according to an embodiment;
Figure 5 is a schematic illustrating a method of medical image processing according to an embodiment;
Figure 6 comprises three images of two-dimensional medical scans in accordance with an embodiment.
Figures 7a, 7b and 7c shows graphs of signed distance in three-dimensional space;
Figure 8 is a schematic illustrating a method of medical image processing according to an embodiment;
Figure 9 is a schematic illustrating a method of medical image processing according to an embodiment;
Figure 10 is a schematic illustrating a method of medical image processing according to an embodiment; and
Figure 11 shows three images used to perform experiments that compare the performance of different embodiments.

**Detailed description**

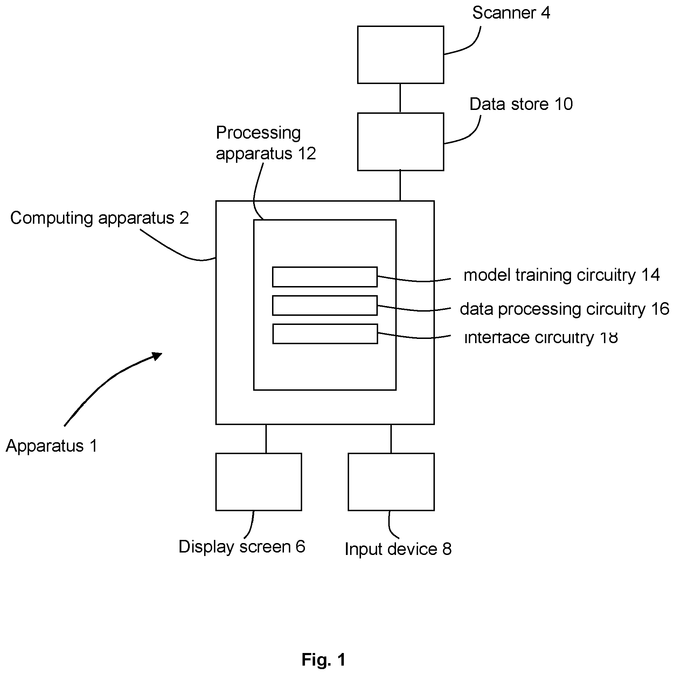

A data processing apparatus 1 according to an embodiment is illustrated schematically in Figure 1. In the present embodiment, the data processing apparatus 1 is configured to process medical data. In other embodiments, the data processing apparatus 1 may be configured to process any other appropriate data.

The data processing apparatus 1 comprises a computing apparatus 2, which in this case is a personal computer (PC) or workstation. The computing apparatus 2 is connected to a display screen 6 or other display device, and an input device or devices 8, such as a computer keyboard and mouse.

The computing apparatus 2 is configured to obtain data sets from a data store 10. The data sets have been obtained or generated using any suitable apparatus or from any suitable source.

In some embodiments, at least some of the data can include, or can be determined from, medical imaging data, for instance obtained using a scanner 4. The scanner 4 may be configured to generate medical imaging data, which may comprise two-, three- or four-dimensional data in any imaging modality. For example, the scanner 4 may comprise a magnetic resonance (MR or MRI) scanner, CT (computed tomography) scanner, cone-beam CT scanner, X-ray scanner, ultrasound scanner, PET (positron emission tomography) scanner or SPECT (single photon emission computed tomography) scanner. The medical imaging data may comprise or be associated with additional conditioning data, which may for example comprise non-imaging data.

The computing apparatus 2 may receive data from one or more further data stores (not shown) instead of or in addition to data store 10. For example, the computing apparatus 2 may receive medical image data from one or more remote data stores (not shown) which may form part of a Picture Archiving and Communication System (PACS) or other information system.

Computing apparatus 2 provides a processing resource for automatically or semiautomatically processing the data. Computing apparatus 2 comprises a processing apparatus 12. The processing apparatus 12 comprises model training circuitry 14 configured to train one or more models, data processing circuitry 16 configured to apply trained model(s) and to perform other processes, and interface circuitry 18 configured to obtain user or other inputs and/or to output results of the data processing.

In the present embodiment, the circuitries 14, 16, 18 are each implemented in computing apparatus 2 by means of a computer program having computer-readable instructions that are executable to perform the method of the embodiment. However, in other embodiments, the various circuitries may be implemented as one or more ASICs (application specific integrated circuits) or FPGAs (field programmable gate arrays).

The computing apparatus 2 also includes a hard drive and other components of a PC including RAM, ROM, a data bus, an operating system including various device drivers, and hardware devices including a graphics card. Such components are not shown in Figure 1 for clarity.

The data processing apparatus 1 of Figure 1 is configured to perform methods as illustrated and/or described in the following.

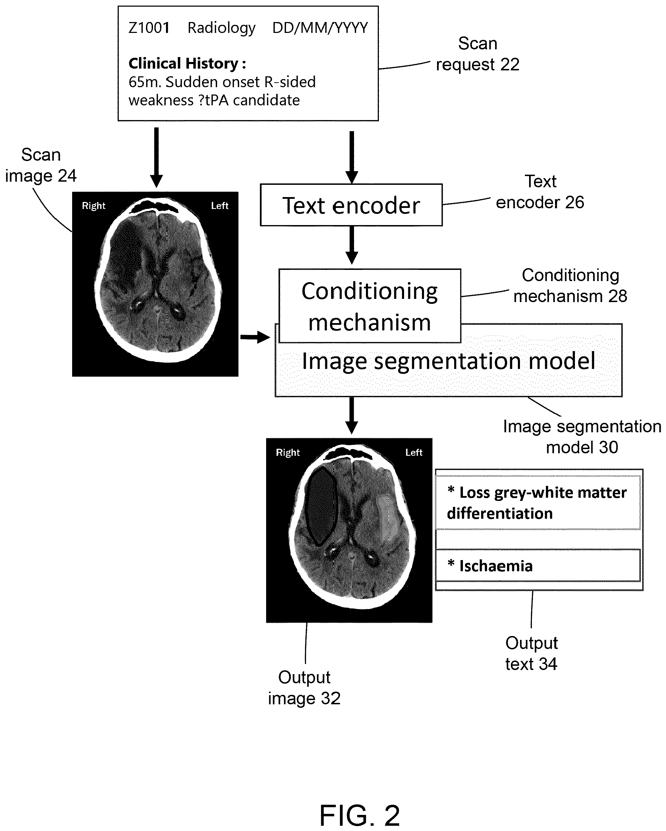

Figure 2 is a schematic representation of a method of medical image processing according to an embodiment. Figure 2 shows a scan request 22 containing textual data including the clinical history of a patient's symptoms. A scan image 24 is obtained using a suitable imaging technology such as X-ray, MRI and CT scans. The scan request 22 is provided to a text encoder 38, in the form of a text encoder network which may comprise a transformer in this embodiment. The text encoder is also referred to as the auxiliary trained machine learning model. The text encoder 26 is configured to generate embeddings for tokens that represent text of the scan request 22.

The text may comprise, for example, any relevant clinical information, for example relating to clinical presentation, the patient's condition, medical history or family history, or relating to a property of the scan, or a measurement parameter of the scan, any other investigation results, findings from physical examination, current medications or other treatments, risk factors for potential diagnoses, or other relevant information to the presence/absence and nature of pathology that is visible in images. For example, scan requests often contain information about the laterality, distribution and type of symptoms (e.g. clinical history), which correspond to the laterality and distribution of the pathology (e.g. stroke lesion). Some specific examples, purely for illustrative purposes, are as follows: "R-sided arm weakness. ?tPA candidate.", "Sudden onset dizziness and vertigo ?POCS ?cerebellar stroke", "Left facial droop. Thrombolysis candidate. Exclude bleed please." The scan request text may contains location information regarding laterality but also symptom body regions (face, arm, etc.) which has a correspondence with particular regions of the brain or other region. It is a feature of certain embodiments that such text can be injected text to a lesion segmentation network or other segmentation model as conditioning information to improve segmentation performance.

The encoded text is provided to a conditioning mechanism 28 and the scan image 24 is provided to an image segmentation model 30. The conditioning mechanism 28 guides the image segmentation model 30 to a particular location or locations in the scan image 24 based on the test of the scan request 22. This location or locations are also referred to as the target processing region. The conditioning mechanism may comprise an INSIDE mechanism or a FiLM mechanism, or any other suitable conditioning mechanism. The FiLM mechanism used may be as described in FiLM: Visual Reasoning with a General Conditioning Layer, Perez et al, arXiv: 1709.07871, 2017. The INSIDE mechanism may be as described in United States Patent Application No. 16/992,466, the contents of which are hereby incorporated by reference in their entirety. The image segmentation model processes the image and text information received to generate an output image 32 and output text 34. The image segmentation model is also referred to as the main machine learning model.

While this embodiment describes using text from a scan request 22, text from any relevant source may be used as an input to the text encoder 26.

In other embodiments the method works in the latent space of the image segmentation model, such that the conditioning mechanism uses the text data to specify, for example provide increased attention to, selected features or characteristics in the latent space. The selected features or characteristics may be referred to as a selected space to which attention is given. For instance, in one example a symptom relating to the right side of the body (e.g. "patient presented with right-sided weakness") would indicate a lesion on the contralateral (left) side of the brain, and therefore if the text mentions a symptom and its laterality, the laterality information may be extracted in the text encoder and passed into the image segmentation network, mapping to enhanced attention on the contralateral (left) side. Sometimes the symptom itself can be mapped to a specific brain region e.g. "vertigo" to the cerebellum ("patient reports experiencing vertigo for the past 4 hours").

In the embodiment of Figure 2, the image segmentation model 30 is a trained convolutional neural network (CNN) that is trained, for example using model training circuitry 14 or by any other remote or local training process. The model may be pre-trained and provided to apparatus 1 in some embodiments, or may be stored remotely and accessed by the apparatus 1 via a network or other communication link. The training is performed in this embodiment using annotated training sets of medical image data comprising medical image data, for example either two dimensional data or three dimensional data, representing the anatomical region that is under consideration, and for each data set the annotations provide an identification of a particular anatomical feature or pathology of interest that is the subject of the model training process. The model training process is intended to train the model so that the trained model can be applied subsequently to new data sets, for example by the data processing circuitry 16, to obtain annotation of the anatomical feature or pathology of interest in each of the new data sets or to perform any other desired task. Such new data sets may for example be data sets obtained by the scanner 4.

The annotations included in the annotated training set may, for example comprise labels used to represent segmentations of the image data (for example, which pixels or voxels, or regions of pixels or voxels, correspond to the anatomical feature or pathology of interest).

After receipt of the annotated training sets of medical image data they are passed by the interface circuitry 18 to the model training circuitry 14. In the embodiment of Figure 1, the model training circuity 4 provides a convolutional neural network (CNN) and trains the CNN on the annotated training sets of medical images.

The CNN of the embodiments of Figure 2 has a structure or architecture based generally on that of any suitable known CNN and includes a plurality of layers including convolutional layers, fully connected layers and pooling layers together with input and output layers. Any other suitable layers may also be included in accordance with known CNN methods.

In accordance with known techniques, for a particular layer, feature maps are generated as an output from the processes performed by the preceding layer, and the feature map is provided as an input to said layer. The output of that layer, e.g. in the form of a feature map, is then provided as an input to the next layer of the CNN. Each layer can have any suitable number of input channels and output channels. At each layer, any suitable desired processes may be performed, for example filters/convolutions, pooling, ReLU processes or any other desired processes. In embodiments, any suitable number of layers can be provided, and any suitable number and arrangement of layers of each type, constrained only, for example, by the requirements of the particular CNN techniques and architectures being used.

It is a feature of the embodiment of that it includes the conditioning mechanism, for example the INSIDE mechanism or FiLM mechanism, which may in the form of auxiliary network or other model or algorithm in addition to the main CNN. The auxiliary network may be another CNN or any other suitable type of neural network, deep learning algorithm or alternative trainable or other model.

The auxiliary network provides conditioning data that can be used to restrict or influence the space of plausible outputs of the main CNN (for example, the plausible outputs for a task of identifying a particular anatomical feature of interest in each set of image data).

It can be understood that such conditioning data can be relevant to size, location or other characteristics of an anatomical feature of interest.

The output of the conditioning mechanisms can provide scale (y) and shift (fJ) parameters as outputs that are provided as inputs to processes at layers of the model. The scale and shift parameters are used to transform an image, Fc, (e.g. training image data set(s) or a feature map derived from preceding layers of the CNN) in accordance with a batch normalisation process. Any suitable batch normalisation process may be applied in the CNN, for example a batch normalisation process as described in Batch Normalization: Accelerating Deep Network Training by Reducing Internal Covariate Shift, Ioffe et al, 2015, arXiv.1502.03167 or US2016217368.

As part of the processes a separate scale ($\gamma$) and shift ($\beta$) factor may be applied to each channel at the relevant layer(s) of the CNN allowing resulting individual feature maps to be amplified or suppressed, and thus affecting the final prediction of the CNN. However, in general such batch normalisation processes do not provide the flexibility to adjust channels in dependence on spatial position, instead the scale and shift factors modify the whole feature map.

Spatially dependent conditioning in dependence on non-image data is also provided at a layer or layers of the CNN. In particular, in embodiments, a spatially dependent filter is generated that provides an attention mechanism based on a differentiable function (for example, a Gaussian) that may, for example, be provided prior to applying feature-wise transformation.

The filter has the effect of limiting a region of the medical image data such that more attention (for example, more weight or importance) is given to that spatial region of the image data in training the model. For example, if for a subject having particular values of non-image parameters the anatomical feature of interest may be more likely to be found in a particular spatial region of an image (for example, an aligned and/or normalized image) then the filter, acting as an attention function, may ensure that more attention is given to that region of the image when training the CNN or other model to label (for example, segment) the anatomical feature of interest in sets of image data.

In embodiments the filter may be generated as a product ( $a = a_1 a_2^T$ ) of two Gaussian vectors and is then integrated into a conditional instance normalisation layer. In other embodiments there could be three vectors for 3D scan data, for example CT or MRI scan data, which could also be combined via a multiplication operation.

The parameter values that determine the shape and position of the Gaussians, for example values for the peak position and variance of each Gaussian, are determined in the embodiment as an output of the conditioning mechanism.

The filter, acting as an attention function, generated by the auxiliary network can be shared across feature maps or applied one per channel when training the CNN. For example, Gaussians or other filters with different parameter values (e.g. peak position, variance) may be used for different feature maps and/or channels at one or more layers when training the CNN. The values of the parameters of the filters to be applied as attention functions for the different feature maps and/or channels in question can be learned separately by the conditioning mechanism. Alternatively, in some embodiments the same filter with the same parameter values can be used as an attention function for all relevant channels and/or more than feature map.

Although the Gaussian filter, acting as an attention function, may be applied at a single layer of the CNN, in other embodiments the filter, acting as an attention function, can be applied at more than one different layer of the CNN, or different filters obtained from different conditionings, each acting as an attention function, can be applied at different layers of the CNN.

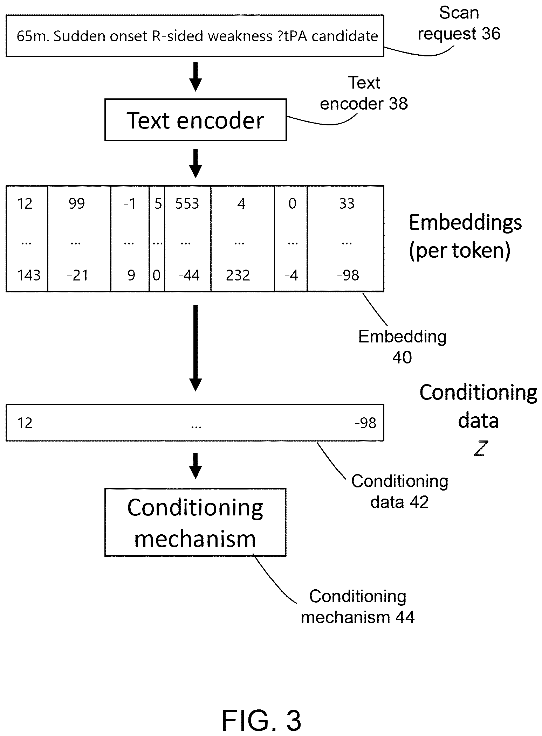

Figure 3 illustrates details of an embodiment of the text encoder 38. A scan request 36 that comprises textual data is provided to the text encoder 38. The text encoder outputs an embedding for tokens representing the text of the scan request 36. Word embedding in Natural Language Processing (NLP) is a technique where individual words are represented as real-valued vectors in a lower-dimensional space and captures inter-word semantics. Linguistic representations may be broken up into tokens where the token may represent a paragraph, a sentence, a word or even elements within words. Each token is represented by a real-valued vector with a plurality of dimensions. In this embodiment, each word of the scan request 36 is considered as one token. The text encoder 38 may use a variety of text encoding models including bag-of-words tokenisers followed by a simple embedding mechanism and pre-trained transformer models such as PubMedBert. The embedding 40 obtained by the text encoder 38 is shown in Figure 3 for one token.

The embeddings 40 are combined, for example by concatenation, into a vector Z that comprises conditioning data 42. The conditioning data 42 is provided to the conditioning mechanism 44. The conditioning data 42 is used to control the spatial attention of the conditioning mechanism 44.

One example of segmentation that is guided by the text encoder 38 is that of laterality, which may be explicitly stated in the scan request 36. In the present embodiment, the scan request 36 contains the text 'R-sided' which the encoder is expected to extract and use to guide the conditioning mechanism 44 and ultimately, the image segmentation model. The text encoder 38 may also, for example, be useful in applications where the scan request 36 or other available text contains implicit information that may influence the selection of a target processing region. In an embodiment, the text encoder 38 can learn how to identify and respond to a type of pathology, such as using 'hollow sphere attention' when fractures are located in the skull. The encoder may be able to identify and respond to a type of symptom, such as applying attention to Broca's area when the input text describes a speech disturbance. The encoder may be able to identify and respond to the location of symptom, such as applying attention to the abdominal area when the input text describes abdominal pain. The encoder may be provided with reports describing the patient's medical history and pathologies. The encoder may be able to use the shape or location of pathologies in previous scans to guide the image segmentation model for a new scan.

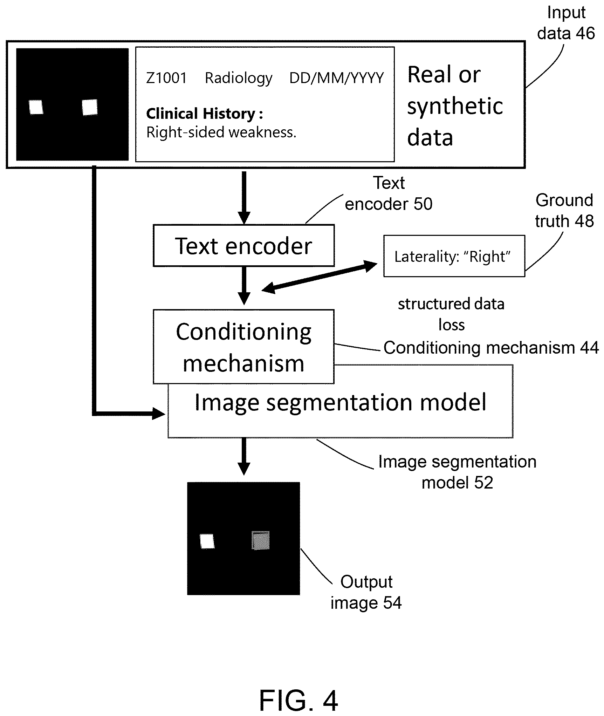

In another embodiment, that may be provided separately, known structured ground truth data can be used to add data supervision at the output of the text encoder 50. Figure 4 shows input data 46 comprising image and text information. The input image can be seen to have two main features, one in the right half of the scan and the other in the left half of the scan. The accompanying text describes the patient's clinical history and recites "Right-sided weakness".

The text in the input data 46 is provided to the text encoder 50 which may comprise a transformer. The text encoder 50 generates embeddings for tokens that represent text of the input data 46. The ground truth 48 information is used to supervise the output of the text encoder 50. The supervision mechanism may, for example, comprise a standard classification task or may comprise a cross-entropy loss based mechanism. Any other suitable supervision mechanism may be used in some embodiments.

The ground truth 48 in this embodiment recites "Laterality: "Right"". The encoded text from the supervised encoding process is provided to a conditioning mechanism 44 and the scan image from the input data 46 is provided to an image segmentation model 52. The conditioning mechanism 44 guides the image segmentation model 52 to a particular location or locations in the scan image based on the text in the input data 46 and text encoding based on the ground truth 48 data. The conditioning mechanism may comprise an INSIDE mechanism or a FiLM mechanism. The image segmentation model processes the image and text information received to generate an output image 54. It can be seen in Figure 4 that the image segmentation model 52 has segmented the feature in the image that is in the right half of the scan.

In another embodiment, synthesised pairs of corresponding images and text can be used to supervise the output of the text encoder. The relationship of text to attention must be similar to the real data but the image may potentially appear quite different. For instance, the laterality relationship may be accurately reproduced in the attention modelling even if the synthetic image does not strongly resemble human anatomy. Note that adding supervision for specific dimensions does not preclude additional useful information being extracted by the network. The structured data supervision may be considered an auxiliary task on the output of the text encoder but the end-to-end training of text encoder with image segmentation model may enable more information to be extracted than just laterality (e.g. information relating to location, appearance of the pathology).

The ground truth used for data supervision in this embodiment was that of the laterality of a symptom. In other embodiments, a variety of other structured ground truths may be used, such as the location of a symptom, the type of a symptom, the type of a pathology and the shape, location or type of pathologies in clinical history.

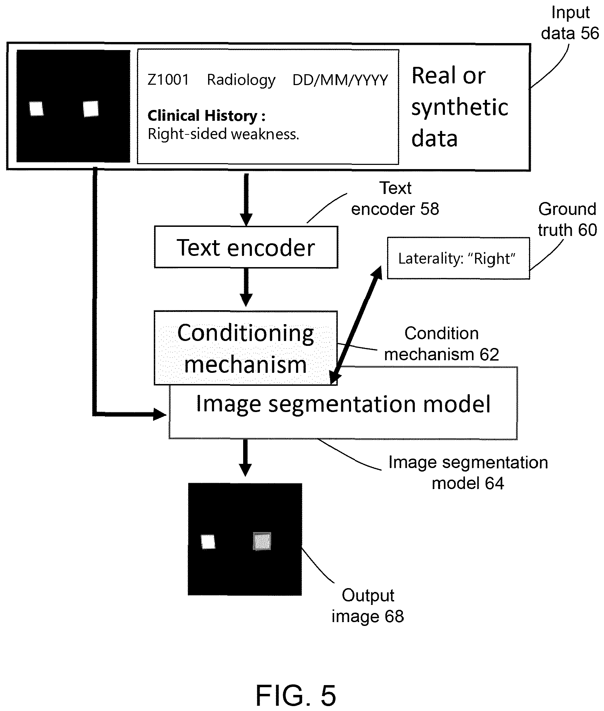

In another embodiment, which may be provided separately, known ground truth data can be used to directly supervise the attention mechanism. The attention vector can be provided to the image segmentation model or may be included in the image segmentation model. Figure 5 shows input data 56 comprising image and text information. The input image can be seen to have two main features, one in the right half of the scan and the other in the left half of the scan. The accompanying text describes the patient's clinical history and recites "Right-sided weakness".

The text in the input data 56 is provided to the text encoder 58 which may comprise a transformer. The text encoder 58 generates embeddings for tokens that represent text of the input data 56 and provides them to the conditioning mechanism 62. The ground truth 60 in this embodiment recites "Laterality: "Right"". Providing the ground truth 60 to the conditioning mechanism 62 can modulate the attention vector of the image segmentation model 64. The mechanism for modulating attention may comprise applying a penalty term to the attention vector based on the ground truth.

Training of the text encoder network, or other auxiliary trained machine learning model, comprises applying a penalty to a loss function in order to train against structured data, and training of the main machine learning model comprises applying a penalty to a loss function in order to train against structured data, in some embodiments.

The scan image from the input data 56 is provided to an image segmentation model 64. The conditioning mechanism 62 guides the image segmentation model 64 to a particular location or locations in the scan image based on the text in the input data 56 and the attention mechanism modulated by ground truth 60 data. The conditioning mechanism may comprise an INSIDE mechanism or a FiLM mechanism. The image segmentation model processes the image and text information received to generate an output image 68. It can be seen in Figure 5 that the image segmentation model 64 has segmented the feature in the image that is in the right half of the scan.

The ground truth user for data supervision in this embodiment was that of the laterality of a symptom. In other embodiments, a variety of other structured ground truths may be used, such as the location of a symptom, the type of a symptom, the type of a pathology and the shape, location or type of pathologies in clinical history.

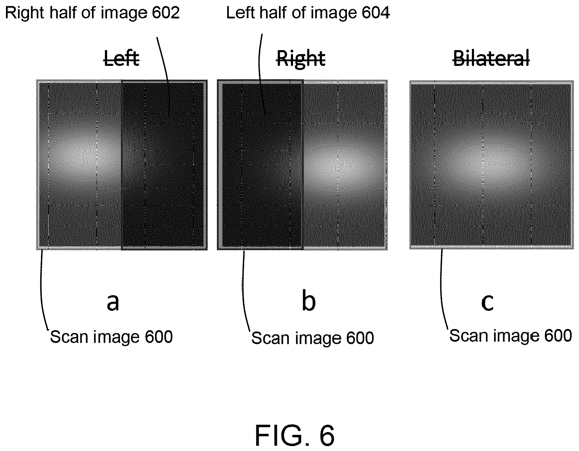

Figure 6 shows three images of medical scan images overlaid with visual indicators of the attention vectors applied to them. The attention vectors in this embodiment are modulated by the mechanism described in the embodiment corresponding to Figure 5 where penalty terms are applied to the attention vector based on the ground truth. Figure 6a shows scan image 600 with the right half of the image 602 penalized for attention vector generation. On the right side of the image, low attention values are rewarded and high attention values are penalised. The vector will exist regardless of the loss function that is placed on its values. This encourages the attention vector to address the left half of the image. Figure 6b shows scan image 600 with the left half of the image 604 penalized for attention vector generation. This encourages the attention vector to address the right half of the image. Figure 6c shows scan image 600 without any penalty applied to the attention mechanism. The attention vector in this case would not be biased towards any particular laterality. While this embodiment was concerned with a two-dimensional scan image, the above techniques can also be applied to a three-dimensional scan image.

In another embodiment which may be provided separately, the conditioning mechanism uses a signed distance function (SDF), or other loss function, for a custom shape rather than controlling the parameters of a distribution such as a Gaussian distribution. It is known that certain appearances and shapes of pathologies are likely to have particular shapes. For example, in Head CT images, fractures are very likely to be found in the skull surface and not in the center of the brain. The SDF can create a smooth decaying gradient around, for example, a hollow sphere for the skull in 3D, or slice the 3D scan to produce and process ring shapes in 2D.

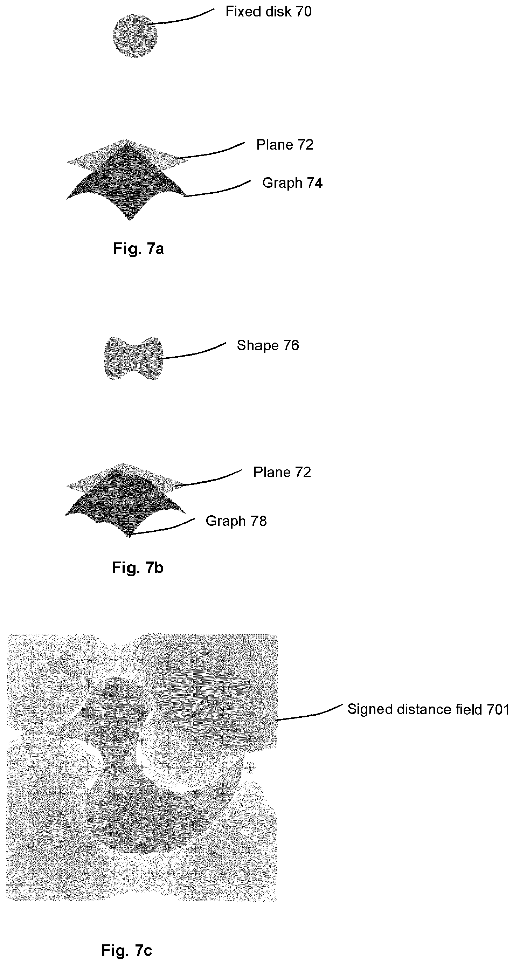

Figure 7a shows a graph 74 of the signed distance between a fixed disk 70 and a point on the plane 72 containing the disk 70. Figure 7b shows a graph 78 of the signed distance between an arbitrary but more complicated shape 76 and a point on the plane 72 containing the shape 76. In figure 7c, a signed distance field 701 is shown as a raster image and used to represent a shape. The field may be stored in this format and used in conditioning the image analysis model.

The text encoder can influence the choice of function that is used for the attention in the conditioning mechanism.

In another embodiment which may be provided separately, the model may be trained on real data alongside augmentations that flip, transform or otherwise modify the attributes of the pathology in order to present the network with direct counter-examples (e.g. counterfactual data) during training that encourage learning of the meaning of the conditioning information. The conditioning input is adjusted corresponding to the attribute flip. By way of example, for the laterality example that has been discussed, there are three possible values (left, right, bilateral) and therefore switching from Left to Right could be considered a flip. However in another example one might modify the type of symptom (e.g. change from "weakness" to "vertigo") and correspondingly change the anatomical location of the attention from cerebrum to cerebellum. In a third example, one might change the timing of the pathology onset (e.g. stroke) from hours to years, and correspondingly change the appearance of the lesion in the image from a faintly darker region to a black region (dead brain i.e. infarct).

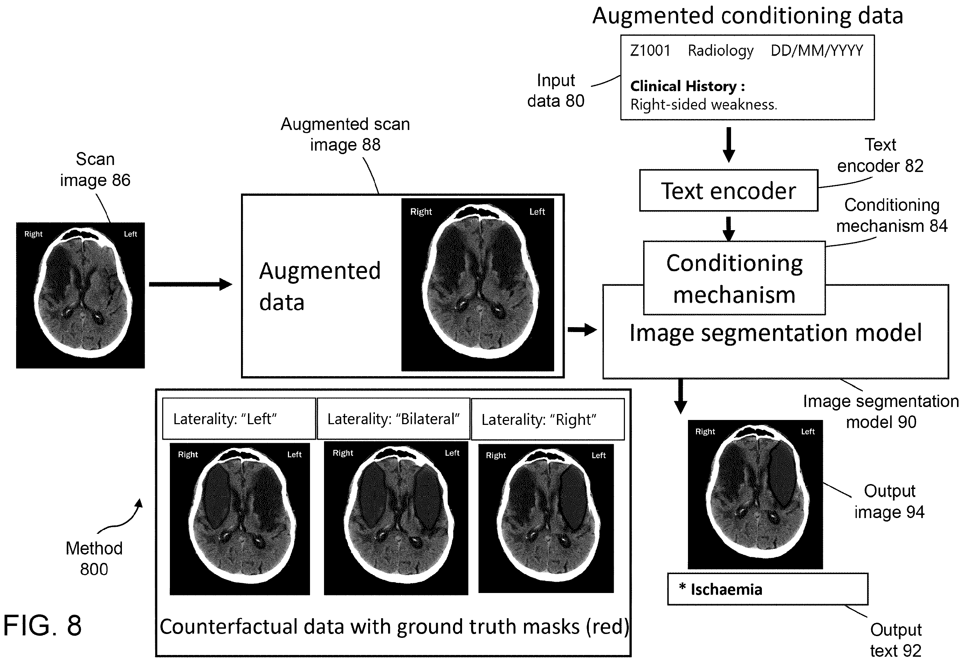

In Figure 8 a method 800 for processing image and text data is shown where the counterfactual augmentations are used. Input data 80 comprising text is provided to a text encoder 82. Word embeddings generated by the text encoder 82 are provided to the conditioning mechanism 84. The scan image 86 is augmented, such as by flipping the attributes of the pathology to obtain the augmented scan image 88. Any other suitable augmentations may be used, for example one might introduce lesions with different location (e.g. part of the brain or other anatomy) or introduce lesions with different appearance (e.g. intensity, texture) of the pathology. It is possible to use simple copy/paste/transform of the original pathology to achieve these patterns, optionally in combination with an aligned atlas to ensure locations map correctly to the input text. The augmented scan image 88 is provided to the image segmentation model 90 which generates the output image 94 and output text 92.

For example, using a "copy, paste" technique the pathology of interest may be copied from the original image (using the segmentation ground truth data) and inserted at a new location. The copy/paste technique involves "copying" the part of the image corresponding to the ground truth pathology label (segmentation) and "pasting" it into a different part of the image, potentially after transforming the appearance e.g. shifting the intensities (brightness) to be lower or higher. This will give 2+ lesions in the image that are candidates for segmentation and means that the network must rely on the text supervision to know which lesion should be segmented. In this way the network may be encouraged to use the text input and not to ignore it. The new location can be determined based on the type of structured data that is used. In method 800, the flipped attribute is laterality, and therefore a pathology is copied or mirrored, and pasted. The laterality is then randomly assigned and the correct ground truth segmentation is then chosen. For instance, the *ground truth segmentation label*, not the mask, may be modified. The augmented image is deliberately the same but it has 2 potential pathologies (left and right). The ground truth mask may be changed to be either a) Left b) Right c) Bilateral and the corresponding conditioning text may be changed to agree with this. Therefore the network learns to encode the information about laterality in the text in order to segment the correct lesion(s).

The training data in various embodiments may comprise at least some training data that represent counterfactual examples or other property that encourage the main machine learning model to use or be more influenced by the conditioning information and/or the output of the auxiliary trained machine learning model.

In another embodiment which may be provided separately, the model may be used in a feedback loop with a clinician, in which the clinician "prompts" the model to give improved segmentations using conversational language. This may require additional training data consisting of user input and prompts.

Any other suitable user input is obtained in other embodiments, and spatial attention mechanism(s) of the main machine learning model are altered in dependence on the user input and/or the text data is augmented or otherwise modified data based on the user input. For example, a clinician may want to inject additional text if the segmentation result is unsatisfactory (perhaps due to sparse clinical history or clinical history that overly narrows attention to one part of the image causing it to ignore others). In some embodiments, the text encoder may be expanded or extended to be/interact with a large language model (LLM) or other suitable trained model. Examples may include any clinical direction as to position & appearance of lesions that we want to segment e.g. "Can you segment the chronic lesion?", "Can you segment any age-related change?", "Can you segment the subdural haemorrhage on the left side?".

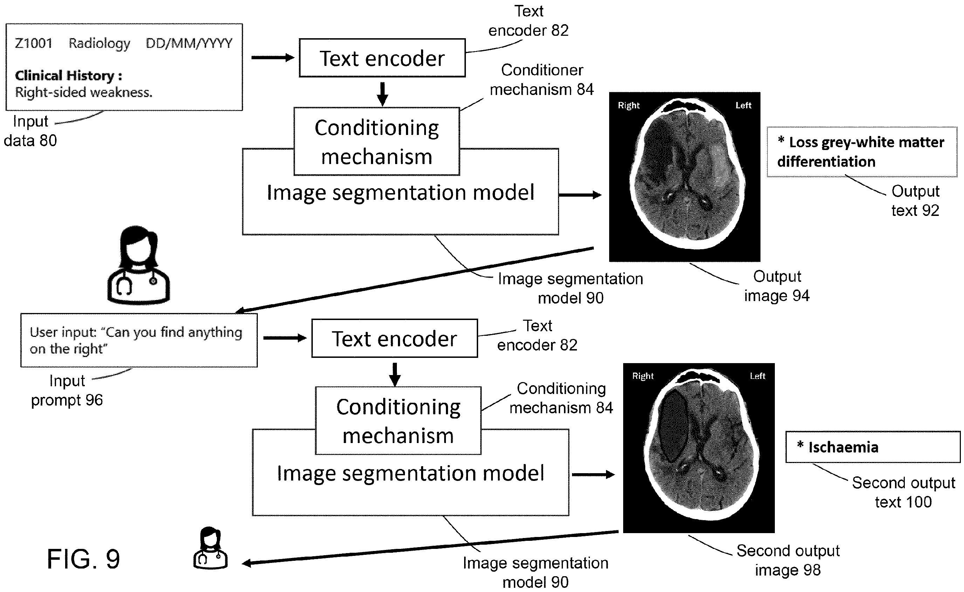

Figure 9 shows two stages of the process. In the first stage, input data 80 is provided to a text encoder 82, followed by a conditioning mechanism 84 and an image segmentation model 90. The image segmentation model 90 generates an output image 94 and output text 92. The user may then provide a further text input to the text encoder in the form of an input prompt 96. In the current embodiment, the input data 80 contained information on a pathology in the right side of the image. The input prompt 96 recites "can you find anything on the left?". The input prompt 96 is processed by the text encoder 82, followed by a conditioning mechanism 84 and an image segmentation model 90. The image segmentation model generates a second output image 98 and second output text 100 on the basis of the input prompt 96.

In another embodiment, which may be provided separately, we can apply a Named Entity Recognition (NER) mechanism to extract entities from the text. The NER mechanism may be an attention layer appended to the text encoder. The encodings from the text encoder for these entities can be extracted and used to condition a separate attention mechanism.

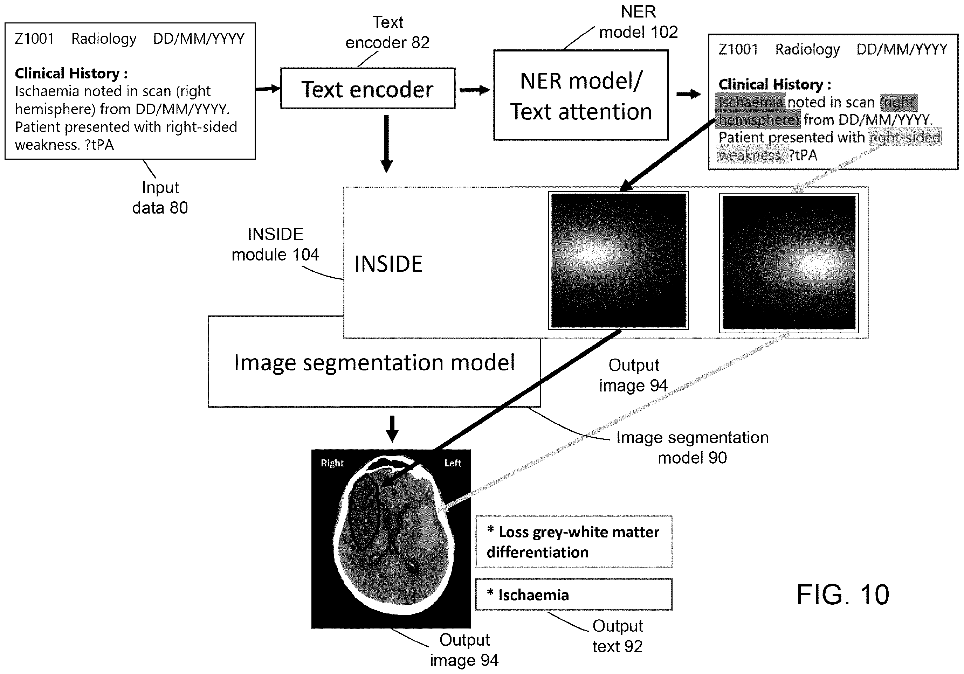

Figure 10 shows input data 80 provided to a text encoder 82. The vector representations of the text obtained by the text encoder 82 are provided to a NER model 102 as well as an INSIDE module 104. The NER model may be a known text classification model (e.g. BERT) or linguistic rules-based algorithm that detects entities. The output can be predictions for the target pathologies and its attributes, so that the attribute information can be used to supervise the attention mechanism(s) to better locate each target pathology(ies). In other embodiments the entities may be used as the conditioning input. This is somewhat similar to the structured data loss approach but it is aimed to extract useful structured data not just as auxiliary supervision but as the actual output that is input instead of text to the imaging model.

Experimental results will now be discussed where the INSIDE mechanism is extended to three-dimensional scans. The experiments also test that laterality can be used as conditioning information for INSIDE. These experiments are performed using structured data only.

It is seen that the model correctly learns the attention for left, right and bilateral inputs. The examples on the right use Gaussian attention. Gaussian attention may be replaced with a signed distance function to learn any shape. For example, the text "?fracture" might cause a hollow sphere attention to be learnt, as this will focus the model on the skull.

Table 1 reports the conditioning data vector 'Z' and the segment task for three different literalities.

*Table 1*

| Input laterality | Conditioning data vector | Segmentation task |
|---|---|---|
| Left | [1, 0] | Left cube |
| Right | [0, 1] | Right cube |
| Bilateral | [1, 1] | Both cubes |

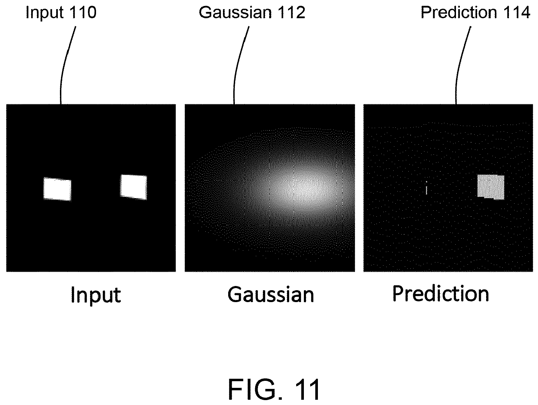

Figure 11 shows the input image 110, the Gaussian attention 112 and the prediction 114 of the image segmentation model 90 for these experiments.

In the first experiment, a model is trained on real data only, to see if adding laterality information improves segmentation accuracy. The results for the first experiment are reported in Table 2. It is seen that the addition of conditioning information as well as the further addition of laterality in the conditioning information, improves the overall Dice score for the process.

*Table 2*

| Model | Conditioning input (z) | Overall Dice Score |
|---|---|---|
| UNet3D | - | 0.721 |
| UNet3D + INSIDE : No conditioning information z (ablation) | Ablation study | 0.726 |
| UNet3D + INSIDE: Laterality input as conditioning information z | Structured laterality data (as in Table 1) | **0.740** |

In a second experiment, as a sanity check that the model is using INSIDE correctly, the real dataset is augmented using the "copy, mirror, paste" technique for laterality in which we have contrived to make the segmentation rely on the conditioning information (since there are 2 lesions and the conditioning information specifies which to segment). We augment both the training and validation data, so we expect the UNet3D model's performance to drop compared to results in the first experiment.

Table 3 reports the results of the second experiment.

*Table 3*

| Model | Conditioning input (z) | Overall Dice Score |
|---|---|---|
| UNet3D | - | 0.683 |
| UNet3D + INSIDE | Structured laterality data (as in Table 1) | **0.742** |

The data used to obtain the results of Tables 2 and 3 comprises a dataset containing 188 Head CT scans with ground truth segmentations for haemorrhages and the surrounding oedema. Volumes are preprocessed by windowing, normalising, and resampling to 2mm resolution. The dataset is split into 85 training volumes (73 with haemorrhage), 20 validation (18 with haemorrhage) and 83 test volumes (69 with haemorrhage). Models were trained on the real data only. One model was trained that contained only the UNet3D. Another model was trained that included the INSIDE conditioning module but as an ablation sensible conditioning information was not input. Finally, another model was trained using the laterality as the input to the conditioning model.

Any suitable trained modes may be used in various embodiments. In the embodiment used to obtain the results of Tables 2 and 3, a 3D UNet encoder-decoder architecture (for example as described in (Ronneberger, Olaf, Philipp Fischer, and Thomas Brox. "U-net: Convolutional networks for biomedical image segmentation." Medical Image Computing and Computer-Assisted Intervention-MICCAI 2015: 18th International Conference, Munich, Germany, October 5-9, 2015, Proceedings, Part III 18. Springer International Publishing, 2015) is used with three downsampling/upsampling stages. Each encoder stage consists of two weight-standardized convolutions (for example as described in Qiao, Siyuan, et al. "Micro-batch training with batch-channel normalization and weight standardization." arXiv preprint arXiv:1903.10520 (2019)) with kernel sizes of 3 and 32, 64, 128 output channels for the three stages respectively followed by Swish activations (for example as described in Ramachandran, Prajit, Barret Zoph, and Quoc V. Le. "Searching for activation functions." arXiv preprint arXiv: 1710.05941 (2017)) and group normalization (for example as described in Wu, Yuxin, and Kaiming He. "Group normalization." Proceedings of the European conference on computer vision (ECCV). 2018). Average 2x2x2 pooling is used for downsampling. The decoder architecture mirrors the encoder in reverse, using transposed convolutional layers for upsampling. A combination of dice and binary cross entropy loss for the 3D UNet model is used, and the model is trained for 400 iterations with a batch size of 2. Adam (for example as described in Reddi, Sashank J., Satyen Kale, and Sanjiv Kumar. "On the convergence of adam and beyond." arXiv preprint arXiv:1904.09237 (2019)) is used with OneCycleLR learning rate schedule (for example as described in Smith, Leslie N., and Nicholay Topin. "Super-convergence: Very fast training of neural networks using large learning rates." Artificial intelligence and machine learning for multi-domain operations applications. Vol. 11006. SPIE, 2019) with maximum learning rate of 0.0001, and early stopping is applied with respect to Dice on the validation dataset (with a patience of 20 epochs).

In a third experiment, a synthetic imaging dataset is used as input to the model with real text data. A text encoder model (PubMedBERT [17]) is added to the same setup used in the experiments described above. The output of the text encoder model feeds into the conditioning module. This setup was trained using a batch size of 8 for 50 epochs with early stopping on the Dice score on the validation dataset (patience of 20). Different learning rates were used for the image and text part of the model ($5e^{-6}$ for text, $5e^{-4}$ for imaging). A real text dataset obtained from the radiology reports was used, associated with the real head CT scans used in the first two experiments. The clinical history/indication field was extracted for each report and mapped to a laterality. For each synthetic image, based on the laterality of its associated ground truth segmentation a real clinical history that matches was picked. Results show that the text input can successfully be processed by the text encoder model to improve the predicted segmentations on this data.

Table 4 reports the results of the third experiment.

*Table 4*

| Model | z | Overall Dice Score |
|---|---|---|
| UNet3D | - | 0.78 |
| UNet3D + INSIDE | Text | **0.90** |

Whilst particular circuitries have been described herein, in alternative embodiments functionality of one or more of these circuitries can be provided by a single processing resource or other component, or functionality provided by a single circuitry can be provided by two or more processing resources or other components in combination. Reference to a single circuitry encompasses multiple components providing the functionality of that circuitry, whether or not such components are remote from one another, and reference to multiple circuitries encompasses a single component providing the functionality of those circuitries.

Whilst certain embodiments are described, these embodiments have been presented by way of example only, and are not intended to limit the scope of the invention. Indeed, the novel methods and systems described herein may be embodied in a variety of other forms. Furthermore, various omissions, substitutions and changes in the form of the methods and systems described herein may be made without departing from the spirit of the invention. The accompanying claims and their equivalents are intended to cover such forms and modifications as would fall within the scope of the invention.

**Claims**

1. A medical image processing apparatus (2) comprising processing circuitry (12) configured to:

   receive medical image data for processing;
   receive text data including clinical information relating to the medical image data;
   specify based on the text data a target processing region of the medical image data or of a space derived from the medical image data; and
   process at least the specified target processing region of the medical image data or of the space derived from the medical image data.

2. The apparatus according to claim 1, wherein the processing of at least the specified target processing region comprises performing a segmentation process to segment a specified anatomical feature and/or pathology.

3. The apparatus according to claim 1 or 2, wherein the processing comprises applying a main trained machine learning model to the medical image data, and the main trained machine learning model is configured to include or receive conditioning information to condition outputs of the main machine learning model such that the outputs depend on both the medical image data and the conditioning information.

4. The apparatus according to claim 3, wherein the processing circuitry (12) is configured to apply an auxiliary trained machine learning model to the text data thereby to specify the target processing region.

5. The apparatus according to claim 4, wherein the auxiliary trained machine learning model comprises a text encoder network that is configured to generate embeddings for tokens that represent text of the text data, and to combine the embeddings into a vector that is or forms part of the conditioning information for the main machine learning model.

6. The apparatus according to claim 4 or 5, wherein the processing circuitry (12) is configured to apply an additional conditioning process so as to ensure or encourage that spatial information in the text data is taken into account by the main machine learning model,
optionally:
wherein the additional conditioning process is such as to alter an attention vector representing spatial attention that is input to or included in the main machine learning model.

7. The apparatus according to claim 6, wherein the additional conditioning process is such as to condition the main machine learning model with a representation of a shape or other property of a pathology or other feature of interest,
optionally:
wherein the additional conditioning process is such as to condition the main machine learning model using a signed distance field or other loss function.

8. The apparatus according to any of claims 4 to 7, wherein the auxiliary trained machine learning model is trained by a training process that comprises applying a penalty to a loss function in order to train against structured data.

9. The apparatus according to any of claims 3 to 8, wherein the main machine learning model is trained by a training process that comprises applying a penalty to a loss function in order to train against structured data;
and/or
wherein the main machine learning model is trained using at least some counterfactual training data and/or using a counterfactual training technique,
optionally:
wherein the training data comprises at least some training data that represent counterfactual examples or other property that encourage the main machine learning model to use or be more influenced by the conditioning information and/or the output of an auxiliary trained machine learning model.

10. The apparatus according to any of claims 3 to 9, wherein the processing circuitry (12) is configured to receive user input, and to alter at least one of spatial attention mechanisms of the main machine learning model in dependence on the user input and/or augment or otherwise modify the text data based on the user input.

11. The apparatus according to any of claims 3 to 10, wherein the processing circuitry (12) is configured to receive user input and to use the user input in a feedback loop that comprises modifying at least one of input to the auxiliary machine learning model, output of the auxiliary machine learning model, and/or input to the main machine learning model based on the user input, and recalculating the output of the main machine learning model.

12. The apparatus according to any preceding claim, wherein the text data comprise at least one radiology report and/or clinician notes or other user notes;
and/or
wherein the image data comprises at least one of magnetic resonance imaging (MRI) data, CT (computed tomography) data, cone-beam CT data, X-ray data, ultrasound data, positron emission tomography (PET) data or single photon emission computed tomography (SPECT) data.

13. A method of image processing apparatus comprising:

   receiving medical image data;
   receiving text data including clinical information relating to the medical image data;
   specifying a target processing region of the medical image data or of a space derived from the medical image data, based on the text data; and
   processing at least the specified target processing region of the medical image data or of a space derived from the medical image data.

14. A processing apparatus configured to train at least one of a main machine learning model or an auxiliary machine learning model based on sets of training image data and associated text data,

   wherein the training of the main machine learning model comprises training the main machine learning model to perform a segmentation based on medical image data and conditioning information received from the auxiliary machine learning model; and
   the training of the auxiliary machine learning model comprises training the auxiliary machine learning model on at least text data to provide conditioning information to the main machine learning model to specify a target processing region.

15. A method of training at least one of a main machine learning model or an auxiliary machine learning model based on sets of training image data and associated text data, the method comprising:

training the main machine learning model to perform a segmentation based on medical image data and conditioning information received from the auxiliary machine learning model; and/or
training the auxiliary machine learning model on at least text data to provide conditioning information to the main machine learning model to specify a target processing region.

Scanner 4

Data store 10

Processing
apparatus 12

Computing apparatus 2

model training circuitry 14

data processing circuitry 16

interface circuitry 18

Apparatus 1

Display screen 6          Input device 8

**Fig. 1**

Z1001    Radiology    DD/MM/YYYY

**Clinical History :**
65m. Sudden onset R-sided
weakness ?tPA candidate

Scan
request 22

Scan
image 24

Text encoder

Text
encoder 26

Conditioning
mechanism

Conditioning
mechanism 28

Image segmentation model

Image segmentation
model 30

* **Loss grey-white matter differentiation**

* **Ischaemia**

Output
text 34

Output
image 32

FIG. 2

65m. Sudden onset R-sided weakness ?tPA candidate | Scan request 36

Text encoder | Text encoder 38

| 12 | 99 | -1 | 5 | 553 | 4 | 0 | 33 |
| ... | ... | ... | ... | ... | ... | ... | ... |
| 143 | -21 | 9 | 0 | -44 | 232 | -4 | -98 |

Embeddings (per token)

Embedding 40

| 12 | ... | -98 |

Conditioning data
$Z$

Conditioning data 42

Conditioning mechanism

Conditioning mechanism 44

FIG. 3

Input data 46

Z1001    Radiology    DD/MM/YYYY

**Clinical History :**
Right-sided weakness.

Real or synthetic data

Text encoder 50

Text encoder

Ground truth 48

Laterality: "Right"

structured data loss

Conditioning mechanism

Conditioning mechanism 44

Image segmentation model

Image segmentation model 52

Output image 54

FIG. 4

FIG. 5

FIG. 6

Fixed disk 70

Plane 72

Graph 74

**Fig. 7a**

Shape 76

Plane 72

Graph 78

**Fig. 7b**

Signed distance field 701

**Fig. 7c**

FIG. 8

FIG. 9

EP 4 421 752 A1

FIG. 10

Input 110  Gaussian 112  Prediction 114

Input  Gaussian  Prediction

# FIG. 11

Europäisches
Patentamt

European
Patent Office

Office européen
des brevets

# EUROPEAN SEARCH REPORT

**Application Number**

EP 24 15 8894

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Andreanne Lemay: "Benefits of Linear Conditioning with Metadata for Image Segmentation", arXiv.org, 26 April 2021 (2021-04-26), XP93168584, Ithaca DOI: 10.48550/arxiv.2102.09582 Retrieved from the Internet: URL:https://arxiv.org/pdf/2102.09582 [retrieved on 2024-05-29] * Abstract * * page 3 - page 5 * | 1-15 | INV. G06V10/26 G06V10/82 |
| A | SHUAILIN LI ET AL: "Shape-aware Semi-supervised 3D Semantic Segmentation for Medical Images", ARXIV.ORG, CORNELL UNIVERSITY LIBRARY, 201 OLIN LIBRARY CORNELL UNIVERSITY ITHACA, NY 14853, 21 July 2020 (2020-07-21), XP081724721, * the whole document * | 1-15 | |
| | | | TECHNICAL FIELDS SEARCHED (IPC) |
| A | ZHAN LI-MING ET AL: "Medical Visual Question Answering via Conditional Reasoning", PROCEEDINGS OF THE 14TH ACM SIGOPS ASIA-PACIFIC WORKSHOP ON SYSTEMS, ACMPUB27, NEW YORK, NY, USA, 12 October 2020 (2020-10-12), pages 2345-2354, XP059440427, DOI: 10.1145/3394171.3413761 ISBN: 979-8-4007-0308-9 * the whole document * | 1-15 | G06V |

The present search report has been drawn up for all claims

| Place of search | Date of completion of the search | Examiner |
|---|---|---|
| The Hague | 30 May 2024 | Ionescu, Tudor |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 992466

- US 2016217368 A

### Non-patent literature cited in the description

- **PEREZ et al.** FiLM: Visual Reasoning with a General Conditioning Layer. *arXiv: 1709.07871,* 2017
- **LOFFE et al.** Batch Normalization: Accelerating Deep Network Training by Reducing Internal Covariate Shift. *arXiv.1502.03167,* 2015
- U-net: Convolutional networks for biomedical image segmentation. **RONNEBERGER ; OLAF ; PHILIPP FISCHER ; THOMAS BROX.** Medical Image Computing and Computer-Assisted Intervention-MICCAI 2015: 18th International Conference, Munich, Germany, October 5-9, 2015, Proceedings, Part III. Springer International Publishing, 05 October 2015, vol. 18
- **QIAO ; SIYUAN et al.** Micro-batch training with batch-channel normalization and weight standardization. *arXiv preprint arXiv:1903.10520,* 2019

- **RAMACHANDRAN ; PRAJIT ; BARRET ZOPH ; QUOC V. LE.** Searching for activation functions. *arXiv preprint arXiv: 1710.05941,* 2017
- **WU ; YUXIN ; KAIMING HE.** Group normalization. *Proceedings of the European conference on computer vision (ECCV),* 2018
- **REDDI ; SASHANK J. ; SATYEN KALE ; SANJIV KUMAR.** On the convergence of adam and beyond. *arXiv preprint arXiv:1904.09237,* 2019
- **SMITH ; LESLIE N. ; NICHOLAY TOPIN.** Super-convergence: Very fast training of neural networks using large learning rates. *Artificial intelligence and machine learning for multi-domain operations applications,* 2019, vol. 11006